# EUROPÄISCHE PATENTSCHRIFT

(11) **EP 1 539 093 B1**
(45) Veröffentlichungstag und Bekanntmachung des Hinweises auf die Patenterteilung: **11.02.2009**
(21) Anmeldenummer: 02769876.0
(22) Anmeldetag: 16.08.2002
(51) Int. Cl.: A61Q 19/00, A61K 8/25, A61K 8/26, A61K 8/27, A61K 8/97, A61K 8/99

(54) **HAUTGLÄTTENDES KOSMETIKUM AUF BASIS VON PFLANZENEXTRAKTEN**
SKIN-SMOOTHING COSMETIC BASED ON PLANT EXTRACTS
AGENT COSMETIQUE A BASE D'EXTRAITS VEGETAUX A EFFET SATINANT DE LA PEAU

(43) Veröffentlichungstag der Anmeldung: 15.06.2005
(73) Patentinhaber: Coty B.V., 2031 CC Haarlem (NL)
(72) Erfinder: GOLZ-BERNER, Karin, MC-98000 Monaco (MC); ZASTROW, Leonhard, MC-98000 Monaco (MC)
(74) Vertreter: Walter, Wolf-Jürgen
(86) Internationale Anmeldenummer: PCT/DE2002/003059
(87) Internationale Veröffentlichungsnummer: WO 2004/017934

(56) Entgegenhaltungen:
- EP-A- 0 962 226
- EP-A- 0 993 822
- DE-C- 4 241 154
- GB-A- 1 066 927
- US-A- 5 925 348
- US-A1- 2002 098 253
- EDITORS R.C. PEPE, J.A. WENNINGER, G.N. MCEWEN: "International Cosmetic Ingredient Dictionary and Handbook, 9th ed., vol. 3" 2002 , THE COSMETIC, TOILETRY AND FRAGRANCE ASSOCIATION , WASHINGTON, D.C. XP002232252 Seite 2628, Spalte 2, Zeile 1-6
- DATABASE WPI Derwent Publications Ltd., London, GB; AN 2001-227311 XP002232253 "Bamboo nutrient facemask preparation" & CN 1 277 839 A ((LIXX-I) LI X), 27. Dezember 2000 (2000-12-27)

## Beschreibung

Die Erfindung betrifft ein Kosmetikum, dessen wirksame Bestandteile bestimmte Pflanzenextrakte sind und das eine sehr gute hautglättende Wirksamkeit aufweist.

Es sind eine Reihe von kosmetischen Produkten bekannt, die eine entzündungswidrige Wirksamkeit für die Haut ausüben. So ist z.B. aus der DE 199 33 857 ein Kosmetikum bekannt mit Pflanzenextrakten aus der Familie der Piperaceae. Weiterhin ist aus einer Vielzahl von Patenten und anderen Veröffentlichungen bekannt, daß Vitamine und Antioxidationsmittel zur Vorbeugung von Alterungserscheinungen und zur Hautpflege eingesetzt werden können. Auch die Neubildung von Hautzellen der oberen Schicht durch Einsatz von Fruchtsäuren (AHA-Säuren; AHA-Effekt) ist bekannt, trägt zur Hauterneuerung bei, ist jedoch wegen der Nebenwirkungen umstritten.

Aus International Cosmetic Ingredient Dictionary and Handbook, 9th ed., Vol. 3 2002 ist das Produkt Sebustop^{®} bekannt, das Cinnamomum cassia, Zingiber off. und Poterium off. enthält.
Die EP 993822 beschreibt die Beschleunigung der Ceramidproduktion durch Zingiber off. und Sanguisorba off.. In der GB 1066927 wird Bambusmilch zur Entfernung von Hautunreinheiten eingesetzt. Der Abstract der CN 1277839 A offenbart ein Gemisch von 4 Pflanzenextrakten, darunter Gesamt-Bambusextrakt und Gesamt-Lotusextrakt für den kosmetischen Einsatz als Gesichtsmaske. Die EP 962226 beschriebt die Verwendung von Wasserkresse-Extrakt u.a. für die Verringerung von Hautirritationen. In der US 2002/0098253 A1 wird eine orale Zusammensetzung mit Hautwirksamkeit beansprucht, die Lotusextrakte enthält.

Die US 5925348 beschreibt eine Anti-Alterungsbehandlung der Haut mit Samenextrakten von Nelumbo nucifera.

Der Erfindung liegt die Aufgabe zugrunde, ein für die Hautglättung poriger oder ungleichmäßiger Haut mit makroskopisch sichtbaren Erhebungen und Vertiefungen geeignetes Kosmetikum ohne irritierende Nebenwirkungen zur Verfügung zu stellen, das in besonders kurzer zeit wirksam wird.

Erfindungsgemäß wird diese Aufgabe durch ein Kosmetikum gelöst, das als Basis Pflanzenextrakte enthält. Es besteht aus 0,01 bis 10 Gew-% eines Extraktes der Blätter und Stengel von Bambusa vulgaris (Bambusmilch),
0,01 bis 5 Gew-% eines Extraktes der Wurzeln von Nymphaea alba,
0,01 bis 5 Gew-% eines Extraktgemisches der Wurzeln von Poterium officinale (anders: Sanguisorba officinalis) und der Wurzeln von Zingiber officinalis und der Rinde von Cinnamomum cassia;
0,01 bis 5 Gew-% eines Extraktes der Gesamtpflanze von Nasturtium officinale R.Br. ;
0,01 bis 5 Gew-% eines Extraktes der Blüten von Nelumbo nucifera Gaertn.;
0,01 bis 20 Gew-% eines Pudergemisches aus Talkum, Bambuspuder, Kaolin und Zinkoxid und
20 bis 95 Gew-% kosmetischen Hilfsstoffen, Trägerstoffen,weiteren Wirkstoffen und Gemischen davon, wobei alle Prozentangaben auf das Gewicht des Kosmetikums bezogen sind.

Bambusmilch wird als Extrakt im wesentlichen aus den Blättern von Bambusa vulgaris gewonnen, wobei Stengelanteile enthalten sind. Durch Extraktion mit wäßrigem Propylenglycol werden sowohl wasserlösliche als auch fettlösliche Bestandteile gewonnen, insbesondere Vitamine, Saponine, Tannine von phenolischen Säuren wie Kaffeesäure und Gallsäure, kieselsäurehaltige Bestandteile, Mineralien, Sterole wie Sitosterol, Stigmasterol und a-Amyrin, sowie Flavonoide. Das Gesamtgemisch, das in wäßrigem Propylenglycol vorliegt und gegebenenfalls auch in einer Trockenform (z.B. sprühgetrocknet) in die kosmetische Zusammensetzung eingebracht werden kann, hat eine hautreinigende, drainierende, adstringierende und entzündungshemmende Wirksamkeit. Bevorzugte Anteile liegen im Bereich von 0,1 bis 5 Gew-%, insbesondere im Bereich von 1 bis 5 Gew-%.

Der Extrakt von Nymphaea alba ist eine orangefarbene Flüssigkeit mit einem pH-Wert von 4,0 bis 6,0 und einer Dichte bei 20 °C von 1,035-1,055. Die Extraktion erfolgt mit einem Wasser:Propylenglycol-Gemisch von 40:60 bis 60:40. Der Wurzelextrakt ist löslich in Wasser und in 60 %igem Ethanol. Durch den Gehalt an Alkaloiden, Flavonoiden und Ascorbinsäure ist eine antibakterielle, tonisierende und auch entzündungshemmende Wirksamkeit erkennbar. Bevorzugte Anteile liegen im Bereich von 0,1 bis 5, insbesondere im Bereich von 1 bis 5 Gew-%.

Das Extraktgemisch der Wurzeln von Poterium officinale und der Wurzeln von Zingiber officinalis und der Rinde von Cinnamomum cassia weist Anteile der Einzelbestandteile im Bereich von jeweils 20-40 Gew-% auf, bezogen auf das Trockengewicht der eingesetzten Rohstoffe. Der Wurzelextrakt von Poterium officinale wird mit einem Gemisch von Wasser und Butylenglycol gewonnen, der Extrakt von Zingiber officinalis und der Rinde von Cinnamomum cassia mit Wasser. Die Wirkung des Extraktgemisches liegt insbesondere in einer antibakteriellen Abwehr und einer Porenverengung. Der Extrakt enthält essentielle öle, Saponoside, Triterpene und Tannine. Er stellt eine transluzide rot-orangene Lösung dar in wäßrigem Butylenglycol, hat einen pH-Wert von 4,0 bis 6,0 und eine Dichte von 1,010 bis 1,040, ist mischbar mit Wasser, Propylenglycol und mit 50 %igem Ethanol und nicht mischbar mit Mineralölen.
Bevorzugte Anteile liegen im Bereich von 0,1 bis 5 Gew-%, insbesondere im Bereich von 1 bis 5 Gew-%.

Der Extrakt von Nasturtium officinale wird aus der Gesamtpflanze mit wäßrigem Propylenglycol gewonnen, hat eine maron-orangene Farbe, eine Dichte bei 20 °C von 1,035 bis 1,055 und ist in Wasser und in Alkohol löslich. Die Wirkung ist antibakteriell und tonisierend. Der Gehalt an Spurenelementen, Carotoniden, essentiellen ölen und Ascorbinsäure scheint die Wirkungsursache zu sein. Bevorzugte Anteile liegen im Bereich von 0,1 bis 5 Gew-%, insbesondere im Bereich von 1 bis 5 Gew-%.

Der Extrakt von Nelumbo nucifera Gaertn. (oder Nelumbium nelumbo Druce) wird aus den Blüten mit wäßrigem Propylenglycol gewonnen, hat eine maron-orangene Farbe, eine Dichte bei 20 °C von 1,02 bis 1,060 und ist in Wasser und Alkohol löslich. Er enthält ein Aminosäuregemisch, Ascorbinsäure und Flavonoide. Bevorzugte Anteile liegen im Bereich von 0,1 bis 5 Gew-%, insbesondere im Bereich von 1 bis 5 Gew-%.

Das Gesamtgemisch in Form des erfindungsgemäßen Kosmetikums zeigt eine über die zu erwartenden Einzelwirkungen hinausgehende porenverengende, hautklärende und insbesondere Hautunebenheiten wie kleine Pickel zurückdrängende Wirksamkeit, die sich durch eine außergewöhnliche Hautglättung innerhalb einer sehr kurzen Zeit darstellt. Besonders der Zeitaspekt in der Wirkungsbreite ist überraschend. Es treten dabei keinerlei Hautirritationen auf, die Wirkung ist sehr hautschonend und die bakterielle Lipase wird deutlich durch die Kombination gehemmt.

Das Kosmetikum kann in einer Ausführungsform weiterhin 0,01 bis 5 Gew-% eines Extraktes von Epilobium angustifolium enthalten bzw. ein Produkt, das diesen Extrakt enthält, z.B. Seborilys® von Greentech, St.Beauzire, Frankreich. Ein solches Produkt wirkt durch 5α-Reductase-Hemmung auf die Enzymaktivität und zeigt eine gewisse anti-androgene Wirksamkeit durch makrocyclische Tannine.

Eine bevorzugte Puderkombination in der o.g. Zusammensetzung besteht aus 8-15 % Talkum, 15-24 % Bambuspuder, 40-58 % Kaolin und 15-25 % Zinkoxid. Dabei kann das Kaolin ein Kaolin gemäß WO96/1758 sein, der mit sphärischen TiO₂- oder SiO₂-Teilchen mit einer Teilchengröße <5 µm modifiziert ist, wobei die sphärischen Teilchen einen Anteil an der Kaolinmischung von 0,5 bis 10 Gew-% haben. Das Präparat erhält dadurch ein sehr weiches Hautgefühl und eine zusätzliche entzündungswidrige Wirksamkeit.

Der eingesetzte Bambuspuder besteht vorzugsweise aus dem pulverisierten Mark von Bambusa arundinacea mit einer bevorzugten mittleren Teilchengröße von etwa 5 µm, wobei etwa 60 % der Teilchen im Bereich von 2-6 µm liegen. Diese spezielle Bambusart wächst in einigen indischen Bergwäldern und eignet sich besonders für die Sebumabsorption und die Texturierung von kosmetischen Produkten. Ein besonders bevorzugtes Produkt ist Greensil^{®} von Greentech, St. Beauzire, Frankreich. Das erfindungsgemäße Kosmetikum kann vorzugsweise nur Bambuspuder allein enthalten.

Der Puderanteil im Gemisch beträgt bevorzugt 0,1 bis 20 Gew-%, insbesondere 5 bis 20 Gew-%.

In einer Ausführungsform der Erfindung kann auch ein Puder aus Methylmethacrylat/Ethylenglycolbismethacrylat-Copolymer zugesetzt werden, der eine durchschnittliche Teilchengröße von etwa 8 µm hat und in Form von makroporösen Kügelchen vorliegt. Der Anteil des PMMA-Puders kann im Bereich von 0,5 bis 10 Gew-% liegen.

Der Puder in der Zusammensetzung hat neben seiner schweißabsorbierenden Wirksamkeit auch eine Abdeckfunktion, indem die Hautunebenheiten von der Sauerstoffzufuhr abgeschirmt werden und dadurch die bakterielle Tätigkeit nicht zusätzlich gefördert wird.

Das erfindungsgemäße Kosmetikum kann weiterhin kosmetische Hilfs- und Trägerstoffe enthalten, wie sie üblicherweise in solchen Zubereitungen verwendet werden, z.B. Wasser, Konservierungsmittel, Vitamine, Farbstoffe, Pigmente, Radikalfänger, Verdickungsmittel, weichmachende Substanzen, Duftstoffe, Alkohole, Polyole wie Glycerin und Propylenglycol und Butylenglycol, Ester oder Ether, Elektrolyte, polare und unpolare öle, Polymere, Copolymere, Emulgatoren, Wachse, Gele, Stabilisatoren, Amine wie Triethanolamin oder Gemische davon.

Pigmente, Pigmentgemische oder Pulver mit pigmentartiger Wirkung, worunter auch solche mit Perlglanz-Effekt zu verstehen sind, können zum Beispiel umfassen Eisenoxide, natürliche Aluminiumsilicate wie Ocker, Titan(di)oxid, Glimmer, Kaolin, manganhaltige Tone wie Umbra und roter Bolus, Calciumcarbonat, Talkum, Glimmer-Titanoxid, Glimmer-Titanoxid-Eisenoxid, Wismutoxychlorid, Nylonkügelchen, Keramikkügelchen, expandierte und nichtexpandierte synthetische Polymerpulver, pulverförmige natürliche organische Verbindungen wie gemahlene Festalgen, gemahlene Pflanzenteile, verkapselte und unverkapselte Getreidestärken sowie Glimmer-Titanoxid-organischer Farbstoff.

Als Ester oder Ether sind zum Beispiel geeignet (INCI-Namen): Dipentaerythrityl hexacaprilate/hexacaprate/tridecyl trimellitate/tridecyl stearate/neopentyl glycol dicaprylate dicaprate, Propylene glycol dioctanoate 5, Propylene glycol dicaprylate 2,30 dicaprate, Tridecyl stearate/neopentyl glycol dicaprylate dicaprate/tridecyl trimellitate, Neopentyl glycol dioctanoate, Isopropyle myristate, Diisopropyl dimer dilinoleate, Trimethylpropane triisostearate, Myristyl ether, Stearyl ether, Cetearyl octanoate, Butyl ether, Dicaprylyl ether, PPG1-PEG9 Lauroyl glycol ether, PPG15 Stearyl ether, PPG14 Butyl ether, PEG20 Stearate, PEG100 Stearate, Fomblin HC25.

In geringen Mengen, z.B. weniger als 5 Gew-%, eingesetzte kosmetische öle können pflanzliche öle sein, wie Calendulaöl, Jojobaöl, Avocadoöl, Macadamianußöl, Rizinusöl, Weizenkeimöl, Traubenkernöl, Kukuinußöl, Distelöl, Nachtkerzenöl, Safloröl oder ein Gemisch mehrerer davon. Es können auch Mineralöle verwendet werden.

Den erfindungsgemäßen Zusamensetzungen können entsprechende wasser- und/oder öllösliche UVA- oder UVB-Filter oder beide zugesetzt werden. Zu vorteilhaften öllöslichen UVB-Filtern gehören 4-Aminobenzoesäure-Derivate wie der 4-(Dimethylamino)-benzoesäure-(2-ethylhexyl)ester; Ester der Zimtsäure wie der 4-Methoxyzimtsäure(2-ethylhexyl)ester, Benzophenon-Derivate wie 2-Hydroxy-4-methoxybenzophenon; 3-Benzylidencampher-Derivate wie 3-Benzylidencampher.

Bevorzugte öllösliche UV-Filter sind Benzophenone-3, Butyl-Methoxybenzoylmethane, Octyl Methoxycinnamate, Octyl Salicylate, 4-Methylbenzylidene Camphor, Homosalate und Octyl Dimethyl PABA.

Wasserlösliche UVB-Filter sind z.B. Sulfonsäurederivate von Benzophenon oder von 3-Benzylidencampher oder Salze wie das Na- oder K-Salz der 2-Phenylbenzimidazol-5-sulfonsäure.

Zu UVA-Filtern gehören Dibenzoylmethan-Derivate wie 1-Phenyl-4-(4'-isopropylphenyl)propan-1,3-dion.

Bevorzugt als Sonnenschutzfilter sind anorganische Pigmente auf Basis von Metalloxiden, wie TiO₂, SiO₂, ZnO, Fe₂O₃, ZrO₂, MnO, Al₂O₃, die auch im Gemisch eingesetzt werden können.

Besonders bevorzugt als anorganische Pigmente sind agglomerierte Substrate von TiO₂ und/oder ZnO, die einen Gehalt an sphärischen und porösen SiO₂-Teilchen aufweisen, wobei die siO₂-Teilchen eine Teilchengröße im Bereich von 0,05 µm bis 1,5 µm haben, und neben den SiO₂-Teilchen andere anorganische teilchenförmige Stoffe mit sphärischer Struktur vorliegen, wobei die sphärischen SiO₂-Teilchen mit den anderen anorganischen Stoffen definierte Agglomerate mit einer Teilchengröße im Bereich von 0,06 µm bis 5 µm bilden (gemäß WO99/06012).

Bevorzugt kann auch ein durch Ultraschallbehandlung hergestelltes Aufschlußprodukt einer Hefe als zusätzlicher Wirkstoff enthalten sein, wobei dieses Aufschlußprodukt Superoxiddismutase (SOD), Protease, Vitamin B₂, Vitamin B₆, Vitamin B₁₂ Vitamin D₂ und Vitamin E enthält. Vorzugsweise enthält es wenigstens 150 IU/ml SOD, Protease und die Vitamine B und D, wobei das Verhältnis SOD:Protease als internationale Einheiten wenigstens im Bereich von 3:1 bis 8:1 liegt.

Besonders vorteilhaft für die Herstellung dieses Aufschlußproduktes als Enzym/Vitamingemisch ist ein Aufschlußverfahren mittels Ultraschall, das in der DE 4241154C1 beschrieben ist.

Die Verwendung des erfindungsgemäßen kosmetischen Präparates kann z.B. erfolgen in Sonnencremes, Sonnengelen, After-sun-Produkten, Tagescremes, Nachtcremes, Masken, Körperlotionen, Reinigungsmilch, Make up's, Lippenstiften, Körperpuder, Augenkosmetik, Haarmasken, Haarspülungen, Haarshampoos, Duschgelen, Duschölen, Badeölen. Die Herstellung derartiger Produkte erfolgt auf eine Weise, wie sie dem Fachmann auf diesem Gebiet bekannt ist.

Die Erfindung soll nachstehend durch Beispiele näher erläutert werden. Alle Angaben erfolgen in Gewichtsprozent, sofern nichts anderes angegeben ist.

### Beispiel 1 Farbige Antipickelcreme

| **Phase A** | |
|---|---|
| Stearyl alcohol | 2,5 |
| Cetaryl Octanoate | 4,3 |
| Mineral oil | 0,6 |
| Pigmente | 3,5 |

| **Phase B** | |
|---|---|
| Wasser | q.s. ad 100 |
| Propylene Glycol | 2,0 |

| **Phase C** | |
|---|---|
| Bambusmilchextrakt (trocken) | 5,0 |
| Nymphaea alba-Extrakt | 3,0 |
| Extraktgemisch Poterium officinale Zingiber officinalis und Cinnamomum cassia | 5,0 |
| Extrakt Nasturtium officinale R.Br. | 0,01 |
| Extrakt Nelumbo nucifera Gaertn. Puderkombination: 10% Talkum, 20% Bambuspuder, 50% Kaolin gemäß WO99/06012, 20% ZnO | 2,0 10,0 |
| Aufschlußprodukt gemäß DE 4241152 aus Saccharomyces cerevisiae | 0,1 |

| **Phase D** | |
|---|---|
| Konservierungsmittel | 0,5 |

Die Phasen A und B werden separat hergestellt, indem die Bestandteile unter Rühren zusammengegeben werden. Beide Phasen werden bei 75 °c ±5 °C zusammengeführt und 20 Min. homogenisiert. Danach wird auf 40°C ±2 °C abgekühlt, die Phase C hinzugegeben und homogenisiert. Im Anschluß daran erfolgt die Zugabe von Phase D.

### Beispiel 2 Vergleich

Die Creme von Beispiel 1 wurde 12 Probanden im Alter von 18 bis 55 Jahren zur Verfügung gestellt. Sie hatten eine unregelmäßige Haut im Bereich Gesicht und Hals mit vereinzelten Pikkeln und großen Poren und hatten bereits regelmäßig andere hautreinigende Kosmetika verwendet.

Die Probanden wurden gebeten, die Creme morgens und abends gleichmäßig und dünn auf die Haut von Gesicht und Hals aufzutragen.

Nach einer Bewertungsskala von 1 bis 5 wurde die Veränderung eingeschätzt.
- 1 =: keine wesentliche Veränderung im Hautbild
- 2 =: geringe Veränderung, Porenverengung
- 3 =: annehmbare Verbesserung in der Hautglättung, Porenverengung, Pickel teilweise etwas eingeebnet
- 4 =: deutliche Verbesserung der Hautglättung, Porenverengung, Pickel größtenteils deutlich reduziert
- 5 =: außerordentlich gute Verbesserung der Hautglättung, Pickel nahezu vollständig eingeebnet, gleichmäßige Hautoberfläche

Zusätzlich wurde noch ein Fragebogen u.a. zu dem Anwendungskomfort und zu Nebenwirkungen ausgefüllt. Von den 12 Probanden bewerteten nach 4 Tagen 2 Probanden mit "3", 6 Probanden mit "4" und 4 Probanden mit "5". Nach 7 Tagen bewerteten 1 Proband mit "3", 7 Probanden mit "4" und 5 Probanden mit "5".

Dies zeigt eine unerwartet schnelle Wirksamkeit bei den meisten Probanden und eine ausgezeichnete Gesamtwirksamkeit nach 7 Tagen, wobei zusätzlich alle Probanden die nebenwirkungsfreie und komfortable Anwendung bestätigten.

Das Ergebnis ist umso überraschender, da die Wirkungen von Einzelkomponenten des Kosmetikums bei einzelnen anderen Probanden zuvor zwar eine gewisse Verbesserung im Hauterscheinungsbild gebracht hatten, dies jedoch erst nach längerdauernder Anwendung von 10 bis 30 Tagen.

## Patentansprüche

1. Hautglättendes Kosmetikum auf Basis von Pflanzenextrakten, **dadurch gekennzeichnet, daß** es enthält
(a) 0,01 bis 10 Gew-% eines Extraktes der Blätter und Stengel von Bambusa vulgaris (Bambusmilch),
(b) 0,01 bis 5 Gew-% eines Extraktes der Wurzeln von Nymphaea alba,
(c) 0,01 bis 5 Gew-% eines Extraktgemisches der Wurzeln von Poterium officinale und der Wurzeln von Zingiber officinalis und der Rinde von Cinnamomum cassia;
(d) 0,01 bis 5 Gew-% eines Extraktes der Gesamtpflanze von Nasturtium officinale R.Br.;
(e) 0,01 bis 5 Gew-% eines Extraktes der Blüten von Nelumbo nucifera Gaertn. ;
(f) 0,01 bis 20 Gew-% eines Pudergemisches aus Talkum, Bambuspuder, Kaolin und Zinkoxid und
(g) 20 bis 95 Gew-% kosmetische Hilfsstoffe, Trägerstoffe, weiteren Wirkstoffe und Gemische davon,
wobei alle Prozentangaben auf das Gewicht des Kosmetikums bezogen sind.

2. Kosmetikum nach Anspruch 1, **dadurch gekennzeichnet, daß** die Extrakte (b) bis (e) in einem Anteil von jeweils 0,1 bis 5 Gew-%, der Extrakt (a) in einem Anteil von 0,1 bis 10 Gew-%, der Puder (f) in einem Anteil von 5 bis 20 Gew-% und die Stoffe (g) mit mindestens 20 Gew-% enthalten sind.

3. Kosmetikum nach Anspruch 1, **dadurch gekennzeichnet, dass** es zusätzlich 0,01 bis 2 Gew-% eines durch Ultraschallbehandlung hergestellten Aufschlussproduktes einer Hefe enthält, wobei dieses Aufschlußprodukt Superoxiddismutase (SOD), Protease, Vitamin B₂, Vitamin B₆, Vitamin B₁₂, Vitamin D₂ und Vitamin E enthält.

4. Kosmetikum nach Anspruch 1, **dadurch gekennzeichnet, daß** es zusätzlich 0,01 bis 5 Gew-% eines Extraktes von Epilobium angustifolium enthält oder ein Produkt, das diesen Extrakt enthält.

5. Kosmetikum nach Anspruch 1, **dadurch gekennzeichnet, daß** der Puder (f) ein Pudergemisch ist, bestehend aus 8-15 % Talkum, 15-24 % Bambuspuder, 40-58 % Kaolin und 15-25 % Zinkoxid, bezogen auf das Gesamtgewicht des Pudergemisches.

6. Kosmetikum nach Anspruch 5, **dadurch gekennzeichnet, daß** der Bambuspuder aus dem pulverisierten Mark von Bambusa arundinacea mit einer mittleren Teilchengröße von 5 µm besteht.

## Claims

1. A skin-smoothing cosmetic based on plant extracts, comprising
(a) 0.01 to 10% by weight of an extract from the leaves and stems of Bambusa vulgaris (bamboo milk);
(b) 0.01 to 5% by weight of an extract from the roots of Nymphaea alba;
(c) 0.01 to 5% by weight of a mixture of extracts from the roots of Poterium officinale, from the roots of Zingiber officinalis and from the bark of Cinnamomum cassia;
(d) 0.01 to 5% by weight of an extract from the entire plant of Nasturtium officinale R.Br.;
(e) 0.01 to 5% by weight of an extract from the flowers of Nelumbo nucifera Gaertn.;
(f) 0.01 to 20% by weight of a powder mixture of talc, bamboo powder, kaolin and zinc oxide; and
(g) 20 to 95% by weight of cosmetic adjuvants, excipients, additional active substances and mixtures thereof,
all percentages being relative to the cosmetic's weight.

2. A cosmetic according to claim 1, wherein said extracts (b) to (e) each make up 0.1 to 5% by weight, said extract (a) makes up 0.1 to 10% by weight, said powder (f) makes up 5 to 20% by weight and the substances (g) with at least 20% by weight.

3. A cosmetic according to claim 1, wherein said cosmetic additionally contains 0.01 to 2% by weight of a product obtained by ultrasonic decomposition of a yeast, which decomposition product comprises superoxide dismutase (SOD), protease, vitamin B₂, vitamin B₆, vitamin B₁₂, vitamin D₂ and vitamin E.

4. A cosmetic according to claim 1, wherein said cosmetic additionally contains 0.01 to 5% by weight of an extract from Epilobium angustifolium or of a product containing said extract.

5. A cosmetic according to claim 1, wherein said powder (f) is a powder mixture consisting of 8-15% talc, 15-24% bamboo powder, 40-58% kaolin and 15-25% zinc oxide, relative to the total weight of the powder mixture.

6. A cosmetic according to claim 5, wherein said bamboo powder consists of the powdered medulla of Bambusa arundinacea having a medium particle size of 5 µm.

## Revendications

1. Agent cosmétique à effet lissant de la peau, à base d'extraits végétaux, **caractérisé en ce qu'**il contient
(a) 0,01 à 10 % en poids d'un extrait de feuille et de tige de *Bambusa vulgaris* (lait de bambou),
(b) 0,01 à 5 % en poids d'un extrait de racines de *Nymphaea alba,*
(c) 0,01 à 5 % en poids d'un mélange d'extraits de racines de *Poterium officinale* et de racines de *Zingiber officinalis* et d'écorces de *Cinnamomum cassia ;*
(d) 0,01 à 5 % en poids d'un extrait de plante complète de *Nasturtium officinale* R. Br ;
(e) 0,01 à 5 % en poids d'un extrait de fleurs de *Nelumbo nucifera Gaertn. ;*
(f) 0,01 à 20 % en poids d'un mélange pulvérulent de talc, de poudre de bambou, de kaolin et d'oxyde de zinc et
(g) 20 à 95 % en poids d'auxiliaires cosmétiques, de véhicules, d'autres substances et leurs mélanges,
tous les pourcentages se rapportant au poids de l'agent cosmétique.

2. Agent cosmétique selon la revendication 1, **caractérisé en ce que** les extraits (b) à (e) sont contenus en une proportion respectivement de 0,1 à 5 % en poids, l'extrait (a) est contenu en une proportion de 0,1 à 10 % en poids, la poudre (f) est contenue en une proportion de 5 à 20 % en poids et les substances (g) sont contenues au moins à hauteur de 20 % en poids.

3. Agent cosmétique selon la revendication 1, **caractérisé en ce qu'**il contient en outre 0,01 à 2 % en poids d'un produit de traitement obtenu par traitement à ultrasons d'une levure, ce produit de traitement contenant de la superoxyde dismutase (SOD), de la protéase, de la vitamine B₂, de la vitamine B₆, de la vitamine B₁₂, de la vitamine D₂ et de la vitamine E.

4. Agent cosmétique selon la revendication, 1, **caractérisé en ce qu'**il contient en outre 0,01 à 5 % en poids d'un extrait d'*Epilobium angustifolium* ou un produit qui contient cet extrait.

5. Agent cosmétique selon la revendication 1, **caractérisé en ce que** la poudre (f) est un mélange pulvérulent constitué de 8 à 15 % de talc, de 15 à 24 % de poudre de bambou, de 40 à 58 % de kaolin et de 15 à 25 % d'oxyde de zinc, par rapport au poids total du mélange pulvérulent.

6. Agent cosmétique selon la revendication 5, **caractérisé en ce que** la poudre de bambou est constituée de la moelle pulvérisée de *Bambusa arundinacea* présentant une taille de particules moyenne de 5 µm.
